(19) Europäisches Patentamt / European Patent Office / Office européen des brevets

(11) **EP 4 349 325 A1**

(12) **EUROPEAN PATENT APPLICATION**
published in accordance with Art. 153(4) EPC

(43) Date of publication:
**10.04.2024 Bulletin 2024/15**

(21) Application number: **22810645.6**

(22) Date of filing: **27.05.2022**

(51) International Patent Classification (IPC):
*A61K 9/127* (2006.01)   *A61K 31/136* (2006.01)
*A61P 35/00* (2006.01)

(52) Cooperative Patent Classification (CPC):
**A61K 9/127; A61K 31/136; A61K 47/24; A61P 35/00**

(86) International application number:
**PCT/CN2022/095446**

(87) International publication number:
**WO 2022/247921 (01.12.2022 Gazette 2022/48)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **28.05.2021 CN 202110593815**

(71) Applicant: **CSPC Zhongqi Pharmaceutical Technology (Shijiazhuang) Co., Ltd.**
**Shijiazhuang, Hebei 050035 (CN)**

(72) Inventors:
• **LI, Chunlei**
  **Shijiazhuang, Hebei 050035 (CN)**
• **LIU, Yanping**
  **Shijiazhuang, Hebei 050035 (CN)**

• **LI, Yanhui**
  **Shijiazhuang, Hebei 050035 (CN)**
• **XIA, Xuefang**
  **Shijiazhuang, Hebei 050035 (CN)**
• **LIANG, Min**
  **Shijiazhuang, Hebei 050035 (CN)**
• **WANG, Caixia**
  **Shijiazhuang, Hebei 050035 (CN)**
• **LI, Chunna**
  **Shijiazhuang, Hebei 050035 (CN)**
• **ZHANG, Jingjing**
  **Shijiazhuang, Hebei 050035 (CN)**
• **WANG, Shixia**
  **Shijiazhuang, Hebei 050035 (CN)**
• **MENG, Hongwei**
  **Shijiazhuang, Hebei 050035 (CN)**

(74) Representative: **Gille Hrabal**
**Partnerschaftsgesellschaft mbB**
**Patentanwälte**
**Brucknerstraße 20**
**40593 Düsseldorf (DE)**

(54) **USE OF MITOXANTRONE HYDROCHLORIDE LIPOSOME IN PREPARATION OF DRUGS FOR TREATING ADVANCED SOLID TUMORS**

(57)   Disclosed is the use of a mitoxantrone hydrochloride liposome in the preparation of drugs for treating advanced solid tumors. A higher therapeutically effective amount of mitoxantrone hydrochloride liposome, for example 8-150 mg/m$^2$, is administered to patients with the advanced solid tumors. Animal test results show that the mitoxantrone hydrochloride liposome can effectively inhibit the growth of various solid tumor transplantation tumors. Clinical research results show that the mitoxantrone hydrochloride liposome can effectively treat advanced solid tumors, and is safe and controllable.

EP 4 349 325 A1

**Description**

The present disclosure claims priority to Chinese Patent Application No. 202110593815.5 filed with China National Intellectual Property Administration on May. 28, 2021 and entitled "USE OF MITOXANTRONE HYDROCHLORIDE LIPOSOME IN PREPARATION OF DRUGS FOR TREATING ADVANCED SOLID TUMORS", which is incorporated herein by reference in its entirety.

PCT Application No. WO2008/080367A1 filed on Dec. 29, 2007 is incorporated herein by reference in its entirety.

**TECHNICAL FIELD**

The present disclosure relates to the field of anti-tumor drugs, and particularly to use of a mitoxantrone hydrochloride liposome for manufacturing a medicament for the treatment of advanced solid tumors.

**BACKGROUND**

Malignant tumors have become one of the major problems seriously threatening the health of Chinese people, and the morbidity and the mortality of malignant tumors have been on a steady rise trend in recent decades. For most solid tumors, local treatment of tumors, such as surgical excision or radiotherapy, is the standard treatment method, but for some patients with locally advanced or metastatic tumors, when the tumors infiltrate widely and are adjacent to or metastasized to important organs, the purpose of "curing" the tumors is often difficult to achieve. Therefore, chemotherapy is still the main treatment method for treating advanced solid tumors. Mitoxantrone is an anthraquinone antibiotic anti-tumor drug, an effective inhibitor of topoisomerase II, and a cell-cycle non-specific drug, which has killing effect on cancer cells proliferating and diffusing and cancer cells not proliferating and diffusing in human body. In addition, mitoxantrone can be inserted into deoxyribonucleic acid through hydrogen bonding, causing the cross-linking and the breakage of a DNA structure; mitoxantrone can also be combined with RNA to interfere the synthesis of over-expressed RNA in tumor cells, thereby achieving the anti-tumor effect. FDA approved NOVANTRONE® (mitoxantrone for injection) in 1987 for the treatment of acute myeloid leukemia, which was then successively approved for indications such as multiple sclerosis, prostate cancer and the like. According to the U.S. labels, in the induction treatment of acute non-lymphocytic leukemia, mitoxantrone is suggested to be used in combination with cytarabine, the recommended dose of mitoxantrone being 12 $mg/m^2$ daily, by intravenous infusion on days 1-3; in the treatment of multiple sclerosis, the recommended dose of mitoxantrone is 12 $mg/m^2$ by intravenous infusion every 3 months; for hormone refractory prostate cancer, the recommended dose of mitoxantrone is 12-14 $mg/m^2$ by intravenous infusion once every 21 days. Meanwhile, clinical studies have shown that mitoxantrone for injection has certain efficacy on a variety of hematological tumors and solid tumors such as malignant lymphoma, breast cancer, lung cancer and the like, but it has very limited clinical application due to serious side effects, such as leukopenia and thrombocytopenia caused by myelosuppression, and severe cardiotoxicity such as palpitation, premature beat, electrocardiogram abnormality and the like.

WO2008/080367A1 discloses a mitoxantrone liposome, which is incorporated herein by reference in its entirety. Studies have shown that a liposome preparation has lower toxicity compared with a common mitoxantrone preparation and can achieve better anti-tumor efficacy at a lower dose. However, the patent documents described above do not discuss the effectiveness and safety of the mitoxantrone liposome in humans. Compared with the common injection, after the drug is prepared into a liposome and is administered to a patient, the *in vivo* behavior of the drug can significantly change. The actual situation is very complex. Whether the administration dose is increased or not and whether the related toxicity is allowed or accepted or not are unpredictable. For example, when a common injection of irinotecan hydrochloride, Camptosar, is clinically used in patients with metastatic cancer of the colon or rectum with recurrent or progressive disease after initial fluorouracil treatment, there are 2 administration regimens. Regimen 1 is once every 1 week at a dose of 125 $mg/m^2$, and 2 weeks of rest after 4 doses; regimen 2 is once every 3 weeks at a dose of 350 $mg/m^2$. The current clinical administration regimen of the liposome injection of irinotecan hydrochloride, Onivyde, is once every 2 weeks at a dose of 70 $mg/m^2$. It can be seen that once the liposome is prepared, the clinical administration dose of the liposome cannot be increased, but is highly reduced. As another example, when a common injection of vincristine sulfate, Oncovin, is used for the treatment of acute leukemia, it is administered at a dose of 1.4 $mg/m^2$ for an adult, once every 1 week; while the liposome preparation of vincristine sulfate, Marqibo, which is also administrated once every 1 week, is administrated at a dose of 2.25 $mg/m^2$, which is 1.6 times of a common injection.

It is well known in the art that liposome is a novel drug carrier that can significantly alter the distribution of encapsulated drugs in the body, and thus, a liposome preparation may have significantly different safe and effective doses for different diseases. For example, Doxil (doxorubicin hydrochloride liposome) has been approved by the FDA for three indications: (1) ovarian cancer, with a recommended dose of 50 $mg/m^2$, administered intravenously once every 4 weeks; (2) Kaposi's sarcoma, with a recommended dose of 20 $mg/m^2$, administered intravenously every 3 weeks; and (3) multiple myeloma, with a recommended dose of 30 $mg/m^2$, administered intravenously on the fourth day after administration of bortezomib. For another example, the starting dose of AmBisome (amphotericin B liposomes for injection) for the treatment of the following indications is: (1) empiric therapy: the recommended dose is 3 mg/kg/day; (2) systemic fungal infection (e.g., Aspergillus, Candida, and Cryptococcus): the recommended dose is 3-5 mg/kg/day; (3) cryptococcal meningitis in HIV-infected patients: the recommended dose is 6 mg/kg/day; and (4) visceral leishmaniasis in immunocompetent patients: 3 mg/kg/day (administered on days 1-5) and 3 mg/kg/day (administered on days 14 and 21); visceral leishmaniasis in immunocompromised patients: 4 mg/kg/day (administered on days 1-5) and 4 mg/kg/day (administered on days 10, 17, 24, 31 and 38), respectively. It is evident that a drug liposome may have different safe and effective doses for different indications. Dosage and dosing parameters should be customized to the disease and patient to maximize efficacy and minimize toxicity or adverse effects for safe and effective treatment of the disease.

The morbidity and the mortality of the advanced solid tumors have been on a continuously rising trend. Chemotherapy is still the main treatment method at present. The efficacy, safety, pharmacokinetic characteristics and the like of the mitoxantrone hydrochloride liposome injection in the advanced solid tumors are not clear at present. The present disclosure preliminarily explores the safety and the effectiveness of the mitoxantrone hydrochloride liposome injection with higher doses in treating the advanced solid tumors so as to provide a basis for clinical application.

## SUMMARY

The present disclosure provides use of a mitoxantrone hydrochloride liposome for manufacturing a medicament for the treatment of advanced solid tumors. Preferably, use of a mitoxantrone hydrochloride liposome as a sole active ingredient for manufacturing a medicament for the treatment of advanced solid tumors.

In some embodiments, the medicament is in a dosage form for injection, including a liquid for injection, a powder for injection, a tablet for injection, and the like. When the medicament is a liquid for injection, the medicament comprises 0.5-5 mg/mL, preferably 1-2 mg/mL, and more preferably 1 mg/mL of the active ingredient, on a basis of mitoxantrone.

The present disclosure further provides a method of treatment of advanced solid tumors, comprising the following steps: administering to a patient with an advanced solid tumor a therapeutically effective amount of a mitoxantrone hydrochloride liposome. Preferably, the mitoxantrone hydrochloride liposome is used alone for treating advanced solid tumors.

The present disclosure further provides use of a mitoxantrone hydrochloride liposome for treating advanced solid tumors.

The present disclosure further provides a mitoxantrone hydrochloride liposome for use in treating an advanced solid tumor in a patient. Preferably, the mitoxantrone hydrochloride liposome is used alone for treating an advanced solid tumor in a patient. In the context of the present disclosure, the mitoxantrone hydrochloride liposome can be in a dosage form for injection, including a liquid for injection, a powder for injection, a tablet for injection, and the like. When the medicament is a liquid for injection, the medicament comprises 0.5-5 mg/mL, preferably 1-2 mg/mL, and more preferably 1 mg/mL of the active ingredient, on a basis of mitoxantrone.

In the context of the present disclosure, the therapeutically effective amount means a dose of mitoxantrone hydrochloride of 8-150 mg/m$^2$, preferably 12-75 mg/m$^2$, more preferably 12-60 mg/m$^2$, more preferably 16-54 mg/m$^2$, more preferably 20-49 mg/m$^2$, and more preferably 24-40 mg/m$^2$, on a basis of mitoxantrone. A dose between any of the 2 dose ranges described above can also be used. For a specific example, 24 mg/m$^2$, 30 mg/m$^2$, 36 mg/m$^2$, 40 mg/m$^2$, 44 mg/m$^2$, 49 mg/m$^2$, 54 mg/m$^2$, 60 mg/m$^2$, and 75 mg/m$^2$, on a basis of mitoxantrone.

In the context of the present disclosure, the route of administration of the mitoxantrone hydrochloride liposome is intravenous administration. Preferably, for each intravenous administration, the dripping administration time of the liposome pharmaceutical preparation is not less than 60 min, preferably 60-120 min, and more preferably 90 $\pm$ 15 min. Preferably, the frequency of administration is once every 3 weeks.

In the context of the present disclosure, the advanced solid tumor is preferably an advanced solid tumor whose conventional therapy is ineffective or that lacks effective treatment, including situations where standard treatment is currently not available, the patient is unable to tolerate the standard treatment, or the like, such as human prostate cancer, advanced lymphoma, relapsed/refractory lymphoma, breast cancer (e.g., advanced recurrent or metastatic breast cancer), ovarian cancer, fallopian tube cancer, colorectal cancer, lung adenocarcinoma, and uterine leiomyosarcoma; preferably fallopian tube cancer, colorectal cancer, lung adenocarcinoma, and uterine leiomyosarcoma.

In the context of the present disclosure, the "therapeutically effective amount" refers to the amount of mitoxantrone hydrochloride liposomes per administration to a patient that is effective to treat, control or alleviate the advanced solid tumor in the patient.

In the context of the present disclosure, unless otherwise specified, the dose is on a basis of mitoxantrone.

In the context of the present disclosure, the mitoxantrone hydrochloride liposome can be manufactured by conventional methods in the art and can be those manufactured by any of the methods disclosed in the prior art, such as the method disclosed in Patent No. WO2008/080367A1.

In some embodiments, the drug or the mitoxantrone hydrochloride liposome has one or more of the following properties:

(i) The mitoxantrone hydrochloride liposome has a particle size of about 30-80 nm, e.g., about 35-75 nm, about 40-70 nm, about 40-60 nm, or about 60 nm;
(ii) Mitoxantrone hydrochloride forms a hardly soluble precipitate with multivalent counterions in the liposome (e.g., sulfate, citrate, or phosphate);
(iii) A phospholipid bilayer in the mitoxantrone hydrochloride liposome contains a phospholipid with a phase transition temperature (Tm) higher than a body temperature, such that the phase transition temperature of the liposome is higher than the body temperature, for example, the phospholipid is selected from hydrogenated soybean lecithin, phosphatidyl choline, hydrogenated yolk lecithin, dipalmitoyl lecithin, distearoyl lecithin, and any combination of the above;
(iv) The phospholipid bilayer in the mitoxantrone hydrochloride liposome comprises hydrogenated soybean lecithin, cholesterol and polyethylene glycol 2000-modified distearoyl phosphatidyl ethanolamine (DSPE-PEG2000);
(v) The phospholipid bilayer in the mitoxantrone hydrochloride liposome comprises hydrogenated soybean lecithin, cholesterol and polyethylene glycol 2000-modified distearoyl phosphatidyl ethanolamine in a mass ratio of about 3:1:1, mitoxantrone hydrochloride forms a hardly soluble precipitate with multivalent acid ions in the liposome, and the particle size of the mitoxantrone hydrochloride liposome in the drug is about 60 nm;
(vi) The mitoxantrone hydrochloride liposome is the mitoxantrone hydrochloride liposome of NMPA Approval No. H20220001.

In the context of the present disclosure, the mitoxantrone hydrochloride liposome has a particle size of about 30-80 nm and comprises: 1) mitoxantrone, an active ingredient, which can form a hardly soluble precipitate with multivalent counterions in the liposome, and 2) a phospholipid bilayer containing a phospholipid with a phase transition temperature (Tm) higher than a body temperature, such that the phase transition temperature of the liposome is higher than the body temperature. The phospholipid with Tm higher than body temperature is selected from one or more of phosphatidyl choline, hydrogenated soybean lecithin, hydrogenated yolk lecithin, dipalmitoyl lecithin, and distearoyl lecithin. The particle size is about 35-75 nm, preferably about 40-70 nm, more preferably about 40-60 nm, and even more preferably about 60 nm. The phospholipid bilayer comprises hydrogenated soybean lecithin, cholesterol and polyethylene glycol 2000-modified distearoyl phosphatidyl ethanolamine in a mass ratio of 3:1:1. The particle size is about 60 nm. The counterion is sulfate. Preferably, the phospholipid bilayer of the liposome comprises hydrogenated soybean lecithin, cholesterol, and polyethylene glycol 2000-modified distearoyl phosphatidyl ethanolamine in a mass ratio of 3:1:1. The particle size is about 40-60 nm. The counterion is sulfate. In the liposome, a weight ratio of HSPC:Chol:DSPE-PEG2000:mitoxantrone is 9.58:3.19:3.19:1.

In the context of the present disclosure, the preparation method of the mitoxantrone hydrochloride liposome is as follows: HSPC (hydrogenated soybean lecithin), Chol (cholesterol) and DSPE-PEG2000 (polyethylene glycol 2000-modified distearoyl phosphatidyl ethanolamine) are weighed in a mass ratio of 3: 1:1 and dissolved in 95% ethanol to obtain a clear solution (i.e., a solution of phospholipids in ethanol). The solution of phospholipids in ethanol is mixed with a 300 mM ammonium sulfate solution, and the resulting mixture is shaken for hydration at 60-65 °C for 1 h to obtain a heterogeneous multivesicular liposome. The particle size of the liposome is then reduced using a micro-jet apparatus. The obtained sample is 200-fold diluted with a 0.9% NaCl solution and then detected with NanoZS. The average particle size of the particles is about 60 nm, with the main peak between 40-60 nm. The ammonium sulfate in the external phase of the blank liposome is then removed using an ultrafiltration device, and the external phase is replaced with 290 mM sucrose and 10 mM glycine in order to form a transmembrane ammonium sulfate gradient. A mitoxantrone hydrochloride solution (10 mg/mL) is added to the blank liposome at a lipid/drug ratio of 16:1, and the drug is encapsulated at 60-65 °C. After 1 hour of incubation, gel size exclusion chromatography determines that the encapsulation efficiency is approximately 100%. The product obtained from this formula is named PLM 60. PLM60 contains HSPC, Chol, DSPE-PEG2000, and mitoxantrone in a weight ratio of 9.58:3.19:3.19:1, and the osmotic pressure of the sucrose-glycine solution is close to the physiological value.

It should be appreciated that a variety of technical details and parameters of the exemplary preparation method described above can be adjusted and determined within the reasonable scope by those skilled in the art. For example, an amino acid alternative to the glycine in the external phase used to form the transmembrane ammonium sulfate gradient includes, but is not limited to, histidine, asparagine, glutamic acid, leucine, proline, and alanine. For another example, a mass ratio of HSPC, Chol, and DSPE-PEG2000 can be appropriately adjusted. As another example, for parameters of the lipid/drug ratio in the preparation of a particular liposome pharmaceutical preparation, those skilled in the art can design, test and ultimately arrive at a suitable lipid/drug ratio to maximize drug-loading rate while reducing drug leakage. For the mitoxantrone hydrochloride liposome preparation of the present application, a wide range of lipid/drug ratios can be used, e.g., as low as 2:1 or as high as 30:1, 40:1, or 50:1 is acceptable, and more suitable lipid/drug ratios may be about (15-20):1, e.g., about 15:1, 16:1, 17:1, 18:1, 19:1, or 20:1. Thus, the several advantageous properties of the mitoxantrone hydrochloride liposome preparation described above are more important, and the methods for achieving these properties are varied.

**Beneficial Effects**

The animal test results of the present disclosure show that the mitoxantrone hydrochloride liposome injection can significantly inhibit the growth of tumors in a dose-dependent manner, the efficacy is far stronger than that of a common mitoxantrone hydrochloride injection, and the duration of the efficacy is significantly superior to that of the latter. Moreover, it proves that the mitoxantrone hydrochloride liposome injection has a better efficacy in the aspects of inhibiting the growth of tumors and/or prolonging the survival rate of tumor-bearing animals compared with the common injection at the same dose. The pharmacokinetic assay shows that compared with the common preparation, the mitoxantrone hydrochloride liposome has the characteristics of significant *in-vivo* long cycles, significant targeting property on tumor tissues, linear kinetic characteristics *in vivo,* and no accumulation *in vivo* in the interval of two administrations. Meanwhile, clinical research results show that the mitoxantrone hydrochloride liposome can effectively treat advanced solid tumors with good efficacy and few adverse effects.

**DETAILED DESCRIPTION**

The technical scheme of the present disclosure will be further illustrated in detail with reference to the following specific examples. It will be appreciated that the following embodiments are merely exemplary illustrations and explanations of the present disclosure, and should not be construed as limiting the protection scope of the present disclosure. All techniques implemented based on the content of the present disclosure described above are included within the protection scope of the present disclosure. Unless otherwise stated, the starting materials and reagents used in the following examples are all commercially available products or can be prepared using known methods.

The following abbreviations are used in the present disclosure.

CR: complete response, specifically defined according to the "Response Evaluation Criteria in Solid Tumors (RECIST1.1)".
PR: partial response, specifically defined according to the "Response Evaluation Criteria in Solid Tumors (RECIST1.1)".
SD: stable disease, specifically defined according to the "Response Evaluation Criteria in Solid Tumors (RECIST1.1)".
PD: progressive disease, specifically defined according to the "Response Evaluation Criteria in Solid Tumors (RECIST1.1)".

$$\text{Objective response rate (ORR)} = (\text{CR} + \text{PR})/\text{total number of evaluable subjects} \times 100\%.$$

$$\text{Disease Control Rate (DCR)} = (\text{CR} + \text{PR} + \text{SD})/\text{total number of evaluable subjects} \times 100\%.$$

**Example 1. Experiment *of In Vivo* Transplanted Tumor in Mice**

Hereinafter, Mit-Inj represents the mitoxantrone hydrochloride injection, and Mit-Lipo represents the mitoxantrone hydrochloride liposome PLM 60.

NU/NU mice were inoculated subcutaneously with PC-3 human prostate cancer cells. On day 28 after inoculation, grouping administration was performed. A solvent (5% glucose injection), Mit-Lipo (CSPC ZHONGQI Pharmaceutical Technology (Shijiazhuang) Co., Ltd., Lot No. 070501), and Mit-Inj (Chongqing Carelife Pharmaceutical Co., Ltd., Lot No. M20050201) were administered intravenously to the mice twice at 6-day intervals (q6d × 2). The experiment was completed on day 26 after the grouping. The experimental results show that the change in the tumor volume of each administration group over time after administration was significantly different ($P < 0.05$). As shown by the relative tumor volume data (see Table 1, which refers to the ratio of the tumor volume at a certain time point to the tumor volume at the starting time point (D0)), the inhibition of tumor growth by the Mit-Inj 2 mg/kg group was maintained only until day 20 after administration, while the Mit-Lipo groups inhibited the tumor growth in a dose-dependent manner and the tumor inhibitory effect was maintained up to the end of the experiment (day 26). At the same dose, the Mit-Lipo 2 mg/kg dose group was significantly different ($P < 0.05$) from the Mit-Inj group at 20-26 days after the first administration. During the study, 1 mouse in the Mit-Inj group died, and no death was observed in the other groups. The results suggested that the mitoxantrone hydrochloride liposome injection has a better tumor growth inhibitory effect, longer duration, and better safety and tolerability compared with the common injection.

Table 1. Inhibition of relative tumor volume of PC-3 human prostate cancer (n = 9, $\bar{x} \pm sd$)

| Time (d) | Solvent control | Mit-Inj | Mit-Lipo | | |
|---|---|---|---|---|---|
| | | 2 mg/kg | 1 mg/kg | 2 mg/kg | 4 mg/kg |
| 4 | 1.7±0.2 | 1.3±0.4[*] | 1.6±0.4 | 1.6±0.3 | 1.7±0.3 |
| 6 | 2.2±0.2 | 1.7±0.4[**] | 1.8±0.5[*] | 1.8±0.5[*] | 1.9±0.4[*] |
| 8 | 2.6±0.4 | 2.0±0.5[**] | 2.1±0.6 | 2.1±0.4[*] | 2.3±0.3[*] |
| 11 | 3.5±0.7 | 2.6±0.4[**] | 2.7±0.7[*] | 2.5±0.5[**] | 2.6±0.3[**] |
| 14 | 4.3±1.0 | 3.2±0.7[*a] | 3.2±1.1[*] | 2.7±0.6[**] | 2.8±0.5[**] |
| 18 | 5.8±1.3 | 4.1±1.0[*a] | 4.2±1.4[*] | 3.4±0.8[**] | 2.8±0.4[**] |
| 20 | 7.0±1.6 | 5.2±1.2[*a] | 5.0±1.6[*] | 3.7±1.1[**▲] | 3.1±0.3[**▲] |
| 22 | 9.0±2.1 | 7.3±1.7[a] | 5.3±1.8[**▲] | 3.7±1.1[**▲▲] | 3.1±0.3[**▲▲] |
| 24 | 10.5±2.9 | 8.3±2.0[a] | 5.6±1.8[**▲] | 4.2±1.5[**▲▲] | 3.5±0.6[**▲▲] |
| 26 | 11.9±2.1 | 9.7±2.4[a] | 7.1±2.8[**] | 4.6±1.5[**▲▲] | 3.6±0.6[**▲▲] |

a: number of animals n = 8; [*]$P < 0.05$, [**]$P < 0.01$, compared with the solvent control group; [▲]$P < 0.05$, [▲▲]$P < 0.01$, compared with the Mit-Inj.

## Example 2. Research Result of Acute Toxicity of Mitoxantrone Liposome Injection

The evaluation of acute toxicity of mitoxantrone hydrochloride liposome and mitoxantrone hydrochloride injection in single administration was carried out using mice and dogs. The results show that after mitoxantrone was prepared into a liposome, the toxicity was significantly reduced compared with that of the mitoxantrone hydrochloride injection.

Table 2. Summary of acute toxicity of the mitoxantrone hydrochloride liposome

| Species and strain | Administration method (vehicle) | Dose (mg/kg) | Drug and Lot No. | Gender and number/ group | Maximum non-lethal dose observed (mg/kg) |
|---|---|---|---|---|---|
| ICR Mouse | Intravenous (5% glucose) | Mit-Inj: 2.52, 3.6, 5.15, 7.35, 10.5, and 15 | Mit-Inj: M-20070104 | 10F 10M | >7.35 |
| | | Mit-Lipo: 25; | Mit-Lipo: 070502; | 10F 10M | >25 |
| Beagle Dog | Intravenous (5% glucose) | Mit-Inj: 0.6, 0.9, 1.34, and 2.0 | Mit-Inj: M-20070104 | 1M | 0.6 |
| | | Mit-Lipo: 0.6, 0.9, 1.34, 2.0, and 3.0; | Mit-Lipo: 070502; | 1M | 2.0 |

## Example 3. Phase I Clinical Trial on Tolerability and Pharmacokinetics of Mitoxantrone Hydrochloride Liposome Injection

A randomized, open-label, single-center and dose-escalation Phase I clinical trial was conducted from August 2011 to June 2013 to evaluate the tolerability of the mitoxantrone hydrochloride liposome injection in patients with advanced tumors. The pharmacokinetic study was conducted concurrently. A total of 6 dose groups, 6 mg/m$^2$, 10 mg/m$^2$, 12 mg/m$^2$, 14 mg/m$^2$, 16 mg/m$^2$, and 18 mg/m$^2$, were set up for the mitoxantrone hydrochloride liposome injection, and 10 mg/m$^2$ of mitoxantrone hydrochloride for injection (common preparation) was used as a control. The drug was administered on the first day of each cycle for up to 3 cycles, each cycle lasting 28 days. 20 subjects were enrolled in the study. 17 subjects were given the mitoxantrone hydrochloride liposome injection, and 3 subjects were given the mitoxantrone hydrochloride for injection (common preparation). The studies showed that at the same dose level (10 mg/m$^2$), the control group (mitoxantrone hydrochloride common injection) showed significantly more frequent and more severe decreases of white blood cells and occurrences of neutrophils than the treatment group. No DLT response occurred in the mitoxantrone hydrochloride liposome treatment group. The preliminary efficacy evaluation results show that the ORR was 22.9% (2/9) and the DCR was 44.4% (4/9).

From February 2014 to September 2017, a Phase I clinical supplemental trial of mitoxantrone hydrochloride liposome, "A clinical trial research on the tolerability of mitoxantrone hydrochloride liposome injection after multiple administrations", was conducted at the Fourth Hospital of Hebei Medical University, which investigated the tolerability and safety of mitoxantrone hydrochloride liposome injection in patients with advanced lymphoma and explored the preliminary efficacy. The research designed a total of 4 dose groups, 18 mg/m$^2$, 20 mg/m$^2$, 22 mg/m$^2$, and 24 mg/m$^2$. The drug was administered on the first day of each cycle for up to 4 cycles with one cycle being 28 days. A total of 17 subjects were enrolled in this study. During the study, only 1 DLT response was observed in the 20 mg/m$^2$ dose group. The DLT cannot be determined. The preliminary efficacy evaluation results show that ORR was 35.3% (6/17) and DCR was 47.4% (9/17).

It can be seen that the change in the formulation improved the safety and tolerability of mitoxantrone. The mitoxantrone hydrochloride liposome injection was safe and well tolerated within the range of 6 mg/m$^2$-24 mg/m$^2$, and the efficacy can be further improved with the increasing dose.

**Example 4. Clinical Trial on Pharmacokinetics of Mitoxantrone Hydrochloride Liposome Injection**

From July 2019, a pharmacokinetic clinical trial (HE071-07) was performed in subjects with relapsed/refractory lymphoma. 18-24 patients were included and randomized into groups in a ratio of 1:1:1 for a total of 4 cycles, Q4W, with the dose of 12 mg/m$^2$, 16 mg/m$^2$, and 20 mg/m$^2$, respectively. The drug concentrations of total mitoxantrone and free mitoxantrone in human plasma at different doses were measured and PK parameters were calculated. PK characteristics of 18 patients were obtained up to May 19, 2020, with the results shown in Table 3 and Table 4.

The $AUC_{0-\infty}$ of total mitoxantrone and free mitoxantrone in plasma in three doses of 12 mg/m$^2$, 16 mg/m$^2$, and 20 mg/m$^2$ increased with the increasing dose. The $AUC_{0-\infty}$ of the total mitoxantrone was 1089.74, 1220.23, and 1435.18 h*μg/mL, respectively. The PK results indicate that the Cmax values of both total mitoxantrone and free mitoxantrone are relatively close in humans after administration of the drug at doses of 16 mg/m$^2$ and 20 mg/m$^2$.

Table 3. Main PK parameters of total mitoxantrone after an intravenous drip of the mitoxantrone hydrochloride liposome

| Total PK parameters | Dose (mg/m$^2$) | | |
| --- | --- | --- | --- |
| | 12 (N=6) | 16 (N=6) | 20 (N=6) |
| $C_{max}$ (ng/mL) | 8890.00±1158.65 (13.0) | 11701.67±1488.36 (12.7) | 11698.33±2016.24 (17.2) |
| $AUC_{0-t}$ (h*μg/mL) | 1068.35±408.37 (38.2) | 1175.28±470.34 (40.0) | 1365.07±214.17 (15.7) |
| $AUC_{0-\infty}$ (h*μg/mL) | 1089.74±412.38 (37.8) | 1220.23±497.48 (40.8) | 1435.18±241.35 |
| $t_{1/2}$ (h) | 85.40±28.53 (33.4) | 68.78±31.51 (45.8) | 81.61±14.82 (18.2) |

Table 4. Main PK parameters of free mitoxantrone after an intravenous drip of the mitoxantrone hydrochloride liposome

| Free PK parameters | Dose (mg/m$^2$) | | |
| --- | --- | --- | --- |
| | 12 (N=6) | 16 (N=6) | 20 (N=6) |
| $C_{max}$ (ng/mL) | 51.57±12.61 (24.5) | 81.43±15.31 (18.8) | 95.30±23.90 (25.1) |
| $AUC_{0-t}$ (h*μg/mL) | 9.66±4.08 (42.2) | 11.74±5.24 (44.6) | 14.25±6.44 (45.2) |
| $AUC_{0-\infty}$ (h*μg/mL) | 10.18±4.34 (42.6) | 12.46±5.78 (46.4) | 15.22±6.14 (40.3) |

(continued)

| Free PK parameters | Dose (mg/m$^2$) | | |
|---|---|---|---|
| | 12 (N=6) | 16 (N=6) | 20 (N=6) |
| t$_{1/2}$ (h) | 125.82±62.06 (49.3) | 72.88±18.77 (25.7) | 90.99±27.54 (30.3) |

**Example 5. Phase II Clinical Trial on Mitoxantrone Hydrochloride Liposome Injection in Advanced Recurrent/Metastatic Breast Cancer**

in patients with advanced recurrent or metastatic breast cancer was performed, with 60 patients with breast cancer included, 30 in each of the treatment group (20 mg/m$^2$ of mitoxantrone hydrochloride liposome injection, administered once every 4 weeks) and control group (14 mg/m$^2$ of mitoxantrone hydrochloride injection, administered once every 4 weeks). The results show that the ORR, DCR, and PFS of the treatment group were all superior to those of the control group. In terms of safety, compared with the common mitoxantrone hydrochloride injection, the incidence of increased cardiac muscle injury marker troponin T in the mitoxantrone hydrochloride liposome injection group was far lower than that of the common mitoxantrone hydrochloride injection (3.3% vs. 36.7%). The overall cardiac safety was good.

**Example 6. Phase I Clinical Trial on Dose Escalation and Dose Expansion of Mitoxantrone Hydrochloride Liposome Injection in Chinese Patients with Advanced Solid Tumors**

Based on the research results described above and referring to the consideration of the initial dose according to Guidelines for Clinical Trial Technology of Anti-tumor Drugs issued by the National Medical Product Administration, "for cytotoxic drugs, the calculation of the initial dose of single administration in phase I clinical trial is, in principle, equivalent to 1/10 of the MTD of the rodent in non-clinical trial or 1/6 of the MTD of the non-rodent, and the unit is mg/m$^2$. Meanwhile, the toxic response and reversibility of the MTD in different species of animals need to be investigated. Generally, the MTD of the animal with the most correlation is selected to estimate the dose. In the case of unknown animal correlation, the MTD of the most sensitive animal is preferably selected for calculation", the Phase I clinical trial on the dose escalation and dose expansion of the mitoxantrone hydrochloride liposome injection (provided by CSPC ZHONGNUO Pharmaceutical (Shijiazhuang) Co., Ltd., the preparation is the same as the drug of NMPA Approval No. H20220001) in Chinese patients with advanced solid tumors was performed. The initial dose of the study was 24 mg/m$^2$. The highest dose of the dose escalation was determined with reference to the toxicity data from non-clinical studies. Pre-clinical animal toxicology experiments showed that the MTD of the mitoxantrone hydrochloride liposome in ICR mice was 25 mg/kg, which was equivalent to the MTD of human of 75 mg/m$^2$; the MTD of beagle dogs was 2 mg/kg, which was equivalent to the MTD of human of 40 mg/m$^2$, so that the MTD of human was predicted to be 40 mg/m$^2$-75 mg/m$^2$ (see Table 5). The highest escalation dose was determined to be 40 mg/m$^2$ based on the consideration of subject safety.

Table 5. Estimated initial and maximum escalation doses

| Test animal | MTD (mg/kg) | Equivalent MTD of human (mg/m$^2$) | MRSD (mg/m$^2$) |
|---|---|---|---|
| ICR mouse | 25 | 75 | 7.5 (1/10) |
| Beagle dog | 2 | 40 | 6.7 (1/6) |

I. Design of experiment

The study is an open and multi-center phase I clinical trial, which is mainly divided into two stages, dose escalation and dose expansion. Subjects with advanced solid tumors are included and administered with the mitoxantrone hydrochloride liposome injection to perform a single drug therapy, which aims to explore the safety and tolerability of the drug, determine the maximum tolerated dose (MTD), observe the pharmacokinetic characteristics and simultaneously evaluate the efficacy (according to RECIST 1.1 criteria).

The trial consists of a screening period, a treatment period and a follow-up period. The screening period lasts 28 days. Subjects sign the informed consent, and complete screening-related tests and baseline assessments. Eligible subjects are screened to enter the treatment period. Subjects are administered with the mitoxantrone hydrochloride liposome injection to perform a single drug therapy in the treatment period. The administration regimen is once every 3 weeks on day 1 of each cycle. Cycle 1 of the dose escalation stage is a DLT observation period. Subjects without occurred DLT events can continue to be dosed in the next cycle at the dose of cycle 1; for subjects with occurred DLT events, the researcher may, as the case may be, reduce a dose level to continue the administration if the subject can tolerate and benefit from the continued treatment according to an assessment; the subject withdraws from the study if the subject cannot tolerate and benefit from the continued treatment according to an assessment. DLT observation is not required in the dose expansion stage. The drug was administered for a total of 6 cycles. For a subject who has completed a 6-cycle administration, if the subject can benefit from and tolerate the continued treatment, the researcher can consult with the sponsor to determine whether the treatment can be continued. During the treatment period, the efficacy is evaluated once every 2 cycles. Pharmacokinetic (PK) blood samples of the subjects are collected at different time points prior to and after the administration as prescribed by the regimen. The relevant examination in all subjects is completed during the study to observe the safety and tolerability. A treatment-ending visit is performed 4 weeks after the last administration, followed by a survival follow-up every 6 weeks (the subject without disease progression requires an efficacy assessment).

1. Dose escalation stage

The initial dose of the mitoxantrone hydrochloride liposome injection is determined to be 24mg/m$^2$, and the higher dose is 30 mg/m$^2$, 36 mg/m$^2$, and 40 mg/m$^2$.

The study follows a "3 + 3" dose escalation regimen. the dose increases to the next dose at a particular dose level, e.g., when no dose limiting toxicity (DLT) occurs in 3 patients in cycle 1; when 1 DLT occurs in cycle 1, then 3 more patients are included; if DLT occurs in 2 or more patients of 3 or 6 patients in cycle 1, then the dose escalation is stopped, and the dose is judged as "a intolerable dose"; the previous dose group of this dose is defined as the maximum tolerated dose (MTD). If the maximum tolerated dose is not explored at the preset maximum dose (40 mg/m$^2$), the researcher consults with the sponsor to determine whether the dose escalation will be continued. Trial doses such as 44 mg/m$^2$, 49 mg/m$^2$, 54 mg/m$^2$, 60 mg/m$^2$, 75 mg/m$^2$, 150 mg/m$^2$, or a dose between any 2 of the dose ranges described above may be selected.

For subjects with DLT events, the investigator may reduce the dose level to continue if the subject is judged to be able to benefit from continued treatment (reduced to 20 mg/m$^2$ if the subject is in the first dose group); the subject will be withdrawn from the study if the subject is judged not to be able to benefit from continued treatment. An increase in dose is not allowed in the same subject.

2. Dose limiting toxicity (DLT)

DLT is defined as an adverse event related to the studied drug within 21 days after the first administration (D1-D21) according to NCI-CTCAE version 5.0.

The specific criteria are as follows.

Hematological toxicity

1) Grade 4 neutropenia is not alleviated for 7 days;
2) Grade 4 thrombocytopenia and reduced hemoglobin;
3) Grade 3 or higher febrile neutropenia is not alleviated for 3 days;
4) Grade 3 thrombocytopenia with bleeding tendency.

Other non-hematological toxicity

1) & grade 2 reduced ejection fraction;
2) Any other ≥ grade 3 non-hematological toxicity, except for the following situations:

(1) Transient (≤ 24 hours) grade 3 fever (> 40 °C), fatigue, headache, and nausea, which returns to grade 1 or baseline after the treatment;
(2) Grade 3 diarrhea, vomiting, electrolyte disturbances (including hypokalemia, hypophosphatemia, hypocalcemia, etc.), which returns to grade 1 or baseline within 48 hours after the treatment.

3. Dose expansion stage

During the experiment, the safety and tolerability of the subject are assessed. 2-4 groups are selected according to the condition of the subject for case expansion (for a tolerable dose group according to the assessment, the dose expansion can be synchronously carried out). 10-20 patients are expanded for each of the selected dose groups. The safety, tolerability, pharmacokinetic characteristics and effectiveness are further observed. The expanded case is not counted in the DLT observation population.

II. Experiment procedure

The study regimen for each subject is as follows: a screening period of -28 to -1 days, a treatment period (6 cycles), a follow-up visit (end of treatment), and a survival follow-up.

After subjects sign the informed consent and complete all baseline tests during the screening period, subjects who meet the inclusion criteria and who do not meet the exclusion criteria will receive a treatment with the mitoxantrone hydrochloride liposome injection. All subjects receive a tumor assessment every 2 cycles during the treatment period to observe the preliminary efficacy of the test drug; meanwhile, the relevant examinations as prescribed by the regimen are completed, and PK (pharmacokinetic) blood samples are collected at different time points prior to and after the administration according to the regimen to observe the safety and pharmacokinetic characteristics. Subjects need to be given a treatment-ending visit 28 ($\pm$7) days after the last administration, followed by a survival follow-up every 6 weeks (the subject without disease progression requires an efficacy assessment).

III. Experiment population

Subjects who meet all of the following inclusion criteria and who do not have any exclusion criteria are eligible for inclusion in the clinical trial.

(I) Inclusion criteria

The subject must meet all of the following criteria:

1. The subject volunteer to participate in the study and sign an informed consent;
2. The age is 18-65 years old, male or female;
3. Patients who have been histopathologically/cytologically diagnosed with advanced solid tumors;
4. A patient with an advanced solid tumor whose conventional therapy is ineffective or who lacks effective treatment according to the judgment of the researcher, including situations where standard treatment is currently not available, the patient is unable to tolerate the standard treatment, or the like;
5. At least one measurable lesion meeting the RECIST 1.1 definition exists at baseline;
6. ECOG score is 0-1;
7. The toxicity of the previous anti-tumor therapy returns to $\leq$ grade 1 or less (except for hair loss, pigmentation, or other toxicity in the study considered to be a non-safety risk to the subject);
8. With a proper organ function, laboratory test values need to meet the following requirements:

- Absolute value of neutrophil count (ANC) & $1.5 \times 10^9$/L (not receiving G-CSF leukogenic treatment within 2 weeks prior to laboratory test);
- Hemoglobin (Hb) & 9.0 g/dL (not receiving infusion of red blood cells within 2 weeks prior to laboratory test);
- Platelets $\geq 100 \times 10^9$/L (not receiving infusion of platelets within 2 weeks prior to laboratory test);
- Creatinine $\leq 1.5 \times$ ULN;
- Total bilirubin $\leq 1.5 \times$ ULN;
- Alanine aminotransferase (AST)/aspartate aminotransferase (ALT) $\leq 3 \times$ ULN;
- Blood coagulation: prothrombin time (PT), International normalized ratio (INR) $\leq 1.5 \times$ ULN;

9. Female subjects are urine or blood HCG negative (except for menopause and hysterectomy); the subject and a partner thereof take effective contraceptive measures from the trial period to 6 months after the end of the last dose.

(II) Exclusion criteria

Subjects who meet any of the following criteria will be excluded from the trial:

1. Severe allergy to mitoxantrone or liposome drugs;

2. A brain or meningeal metastasis subject;

3. Survival < 3 months;

4. Patients with chronic hepatitis B (HBsAg or HBcAb positive and HBV DNA $\geq$ 1000 IU/mL), chronic hepatitis C (HCV antibody positive and HCV RNA is higher than the lower limit of the detection value of the research center), or positive HIV antibody;

5. Suffering from an active bacterial infection or fungal infection or viral infection requiring intravenous infusion within 1 week prior to the first drug administration;

6. Any anti-tumor treatment (such as radiotherapy, targeted therapy, immunotherapy, endocrine therapy, etc.) is received within 4 weeks prior to the first administration; traditional Chinese medicine or Chinese patent medicine treatment approved for tumors as an indication is received within 2 weeks prior to the first administration;

7. Other drugs for a clinical trial are received within 4 weeks prior to the first administration;

8. Patients who have received major surgery within 3 months prior to the first administration, and do not recover after the surgery; or those who plan to receive major surgery during the trial;

9. Serious thrombosis or embolism events as judged by the researcher, such as pulmonary embolism, etc., occur within 6 months prior to the screening;

10. Suffering from other malignant active tumors within 3 years, except for locally treatable cancer that has already been cured, for example, basal or squamous cell skin cancer or *in situ* prostate, cervical, or breast cancer;

11. Cardiac dysfunction, including:

- Long QTc syndrome or QTc interval > 480 ms;

- Complete left bundle branch block, degree II or degree III atrioventricular block;

- Severe, uncontrolled arrhythmia requiring a drug therapy;

- History of chronic congestive heart failure, NYHA $\geq$ grade 3;

- The heart ejection fraction is lower than 50% within 6 months;

- A cardiac valve disease with CTCAE $\geq$ grade 3;

- Uncontrolled hypertension (defined as systolic pressure & 160 mmHg or diastolic pressure & 100 mmHg by measurement under drug control);

- Myocardial infarction, unstable angina pectoris, history of severe pericardial disease, electrocardiographic evidence of acute ischemic or severe conduction system abnormalities within 6 months prior to the screening;

12. Having received doxorubicin or other anthracyclines therapy, and the cumulative doxorubicin dose exceeds 350 mg/m$^2$ (anthracyclines equivalent dose calculation: 1 mg doxorubicin = 2 mg epirubicin = 2 mg pirarubicin = 2 mg daunorubicin = 0.5 mg demethoxydaunorubicin = 0.45 mg mitoxantrone; the cumulative dose is not calculated for a doxorubicin liposome);

13. Women in lactation;

14. Suffering from any severe and/or uncontrollable disease, and other diseases, as judged by the researcher, that may influence the patient to participate in the study (including, but not limited to, diabetes that has not been effectively controlled, kidney diseases requiring dialysis, severe liver diseases, life-threatening autoimmune diseases and hemorrhagic diseases, drug abuse, neurological diseases, etc.);

15. Other circumstances where the subject is not suitable to participate as judged by the researcher.

(IV) Withdrawal/termination criteria

The subject may withdraw from the study or discontinue the study intervention at any stage of the study without any reason. The reason for the subject discontinuation or withdrawal from the study should be recorded in the eCRF. The researcher should perform an ending visit and a evaluation of the subject withdrawing from the study, if possible.

Subjects must withdraw from the study if the following situations occur:

- The subject withdraws the informed consent or the subject or a family member thereof requires withdrawal from the trial;

- The subject has participated in other clinical studies (except for an OS follow-up) during the study.

IV. Results

This study evaluated the efficacy of 19 patients divided into: 7 cases of breast cancer, 7 cases of ovarian cancer and fallopian tube cancer, 3 cases of colorectal cancer, 1 case of lung adenocarcinoma, and 1 case of uterine leiomyosarcoma according to tumor species. The research results show that when the mitoxantrone hydrochloride liposome injection is used, the drug has the functions of sustained release, targeting, toxicity reduction, and efficacy enhancement after the drug enters a human body through intravenous infusion, and it has good clinical application prospect for patients with various advanced solid tumors.

No dose limiting toxicity (DLT) events occurred in the dose escalation studies at both 24 mg/m$^2$ and 30 mg/m$^2$. A total of 25 subjects were enrolled, 15 in the 24 mg/m$^2$ dose group and 10 in the 30 mg/m$^2$ dose group. Adverse effects were mainly hematological toxicity. AEs & grade 3 with the incidence rate > 5% in the 24 mg/m$^2$ group are leukopenia (60%), neutropenia (53%), and decreased platelet count (13%); AEs $\geq$ grade 3 with the incidence rate > 5% in the 30 mg/m$^2$ are neutropenia (30%), leukopenia (30%), decreased platelet count (20%), anemia (10%), and decreased lymphocyte count (10%). In efficacy, the 24 mg/m$^2$ group had at least one efficacy assessment of 12 cases, 2 had PR, 4 had SD, and 6 had PD, showing an ORR of 16.7% and a DCR of 50%; the 30 mg/m$^2$ group had at least one efficacy assessment of 7 cases, 4 had SD and 3 had PD, and showing a DCR of 57.1%. The subject evaluated as SD continued to take the drug, resulting in better efficacy.

The incidence rates of all grades of adverse events of the mitoxantrone hydrochloride liposome injection of this study were compared to those of the common mitoxantrone preparation: leukopenia (76% vs. 87%), neutropenia (64% vs. 79%), decreased platelet count (32% vs. 39%), anemia (60% vs. 75%), and decreased lymphocyte count (4% vs. 72%); in the aspect of cardiac safety, cardiac dysregulation (0% vs. 18.0%), myocardial ischemia (0% vs. 5.0%), and arrhythmia (0% vs. 7.0%). Safety data for mitoxantrone was extracted from the CALGB9182 study. A total of 112 subjects with advanced hormone-refractory prostate cancer were enrolled in the study and were given mitoxantrone as a single agent (12-14 mg/m$^2$) in combination with hydrocortisone treatment.

It can be seen that the mitoxantrone hydrochloride liposome does not increase the haematological toxicity compared to the common mitoxantrone preparation on the premise of improving the dose,, and the incidence rate of the adverse effects related to the heart of the modified mitoxantrone hydrochloride liposome is significantly reduced.

The embodiments of the technical solutions of the present disclosure have been described above by way of example. It should be understood that the protection scope of the present disclosure is not limited to the embodiments described above. Any modification, equivalent replacement, improvement, and the like made by those skilled in the art without departing from the spirit and principle of the present disclosure shall fall within the protection scope of the claims of the present application.

**Claims**

1. Use of a mitoxantrone hydrochloride liposome for manufacturing a medicament for the treatment of advanced solid tumors;
preferably, the advanced solid tumor is an advanced solid tumor for which conventional treatment is ineffective or lacks effective treatment, including situations where standard treatment is currently not available, the patient is unable to tolerate the standard treatment, or the like, such as human prostate cancer, advanced lymphoma, relapsed/refractory lymphoma, breast cancer (e.g., advanced recurrent or metastatic breast cancer), ovarian cancer, fallopian tube cancer, colorectal cancer, lung adenocarcinoma, and uterine leiomyosarcoma; preferably fallopian tube cancer, colorectal cancer, lung adenocarcinoma, and uterine leiomyosarcoma.

2. Use of a mitoxantrone hydrochloride liposome as a sole active ingredient for manufacturing a medicament for the treatment of advanced solid tumors.

3. The use as claimed in claim 1 or 2, wherein a therapeutically effective amount of the mitoxantrone hydrochloride liposome is 8-150 mg/m$^2$, preferably 12-75 mg/m$^2$, more preferably 12-60 mg/m$^2$, more preferably 16-54 mg/m$^2$, more preferably 20-49 mg/m$^2$, and more preferably 24-40 mg/m$^2$, and a dose between any of two dose ranges described above can also be used, in particular 24 mg/m$^2$, 30 mg/m$^2$, 36 mg/m$^2$, 40 mg/m$^2$, 44 mg/m$^2$, 49 mg/m$^2$, 54 mg/m$^2$, 60 mg/m$^2$, and 75 mg/m$^2$, on a basis of mitoxantrone.

4. The use as claimed in any of claims 1-3, wherein the medicament is in a dosage form for injection, including a liquid for injection, a powder for injection, a tablet for injection, and the like; preferably, the medicament is a liquid for injection.

5. The use as claimed in claim 4, wherein the medicament comprises 0.5-5 mg/mL, preferably 1-2 mg/mL, and more preferably 1 mg/mL of the active ingredient, on a basis of mitoxantrone.

6. The use as claimed in any of claims 1-5, wherein the mitoxantrone hydrochloride liposome has a particle size of about 30-80 nm and comprises: 1) mitoxantrone, an active ingredient, which can form a poorly soluble precipitate with multivalent counterions in the liposome, and 2) a phospholipid bilayer containing a phospholipid with a phase transition temperature (Tm) higher than a body temperature, wherein the phospholipid with Tm higher than body temperature is selected from one or more of phosphatidyl choline, hydrogenated soybean lecithin, hydrogenated yolk lecithin, dipalmitoyl lecithin, and distearoyl lecithin;

> or preferably, the liposome has a particle size of about 35-75 nm, preferably about 40-70 nm, and more preferably about 40-60 nm;
> or preferably, the phospholipid bilayer comprises hydrogenated soybean lecithin, cholesterol and polyethylene glycol 2000-modified distearoyl phosphatidyl ethanolamine in a mass ratio of 3:1:1, the particle size is about 60 nm, and the counterion is sulfate;
> or preferably, the phospholipid bilayer of the liposome comprises hydrogenated soybean lecithin, cholesterol, and polyethylene glycol 2000-modified distearoyl phosphatidyl ethanolamine in a mass ratio of 3:1:1, the particle size is about 40-60 nm, the counterion is sulfate, and in the liposome, a weight ratio of HSPC:Chol:DSPE-PEG2000:mitoxantrone is 9.58:3.19:3.19:1.

7. A method of treatment of advanced solid tumors, comprising the following steps:

> administering to a patient with an advanced solid tumor a therapeutically effective amount of a mitoxantrone hydrochloride liposome;
> or preferably, the liposome is used alone for treating advanced solid tumors;
> or preferably, the liposome is in a dosage form for injection, including a liquid for injection, a powder for injection, a tablet for injection, and the like; preferably, the liposome is a liquid for injection; when the liposome is a liquid for injection, the liposome comprises 0.5-5 mg/mL, preferably 1-2 mg/mL, and more preferably 1 mg/mL of the active ingredient, on a basis of mitoxantrone;
> or preferably, a therapeutically effective amount of the mitoxantrone hydrochloride liposome is 8-150 mg/m$^2$, preferably 12-75 mg/m$^2$, preferably 12-60 mg/m$^2$, more preferably 16-54 mg/m$^2$, more preferably 20-49 mg/m$^2$, and more preferably 24-40 mg/m$^2$, and a dose between any of 2 dose ranges described above can also be used, in particular 24 mg/m$^2$, 30 mg/m$^2$, 36 mg/m$^2$, 40 mg/m$^2$, 44 mg/m$^2$, 49 mg/m$^2$, 54 mg/m$^2$, 60 mg/m$^2$, and 75 mg/m$^2$, on a basis of mitoxantrone;
> or preferably, a route of administration of the mitoxantrone hydrochloride liposome is intravenous administration; preferably, for each intravenous administration, a dripping administration time of the liposome pharmaceutical preparation is not less than 60 min, preferably 60-120 min, and more preferably 90 $\pm$ 15 min;
> or preferably, a frequency of administration of the mitoxantrone hydrochloride liposome is once every 3 weeks or 4 weeks.

8. The method as claimed in claim 7, wherein the mitoxantrone hydrochloride liposome has a particle size of about 30-80 nm and comprises: 1) mitoxantrone, an active ingredient, which can form a poorly soluble precipitate with multivalent counterions in the liposome, and 2) a phospholipid bilayer containing a phospholipid with a phase transition temperature (Tm) higher than a body temperature, wherein the phospholipid with Tm higher than body temperature is selected from one or more of phosphatidyl choline, hydrogenated soybean lecithin, hydrogenated yolk lecithin, dipalmitoyl lecithin, and distearoyl lecithin;

> or preferably, the liposome has a particle size of about 35-75 nm, preferably about 40-70 nm, and more preferably about 40-60 nm;
> or preferably, the phospholipid bilayer comprises hydrogenated soybean lecithin, cholesterol and polyethylene glycol 2000-modified distearoyl phosphatidyl ethanolamine in a mass ratio of 3:1:1, the particle size is about 60 nm, and the counterion is sulfate;
> or preferably, the phospholipid bilayer of the liposome comprises hydrogenated soybean lecithin, cholesterol, and polyethylene glycol 2000-modified distearoyl phosphatidyl ethanolamine in a mass ratio of 3:1:1, the particle size is about 40-60 nm, the counterion is sulfate, and in the liposome, a weight ratio of HSPC:Chol:DSPE-PEG2000:mitoxantrone is 9.58:3.19:3.19:1.

9. A mitoxantrone hydrochloride liposome for use in treating an advanced solid tumor in a patient;

or preferably, the liposome is used alone for treating advanced solid tumors;

or preferably, the liposome is in a dosage form for injection, including a liquid for injection, a powder for injection, a tablet for injection, and the like; preferably, the liposome is a liquid for injection; when the liposome is a liquid for injection, the liposome comprises 0.5-5 mg/mL, preferably 1-2 mg/mL, and more preferably 1 mg/mL of the active ingredient, on a basis of mitoxantrone;

or preferably, a therapeutically effective amount of the mitoxantrone hydrochloride liposome is 8-150 mg/m$^2$, preferably 12-75 mg/m$^2$, preferably 12-60 mg/m$^2$, more preferably 16-54 mg/m$^2$, more preferably 20-49 mg/m$^2$, and more preferably 24-40 mg/m$^2$, and a dose between any of 2 dose ranges described above can also be used, in particular 24 mg/m$^2$, 30 mg/m$^2$, 36 mg/m$^2$, 40 mg/m$^2$, 44 mg/m$^2$, 49 mg/m$^2$, 54 mg/m$^2$, 60 mg/m$^2$, and 75 mg/m$^2$, on a basis of mitoxantrone;

or preferably, a route of administration of the mitoxantrone hydrochloride liposome is intravenous administration; preferably, for each intravenous administration, a dripping administration time of the liposome pharmaceutical preparation is not less than 60 min, preferably 60-120 min, and more preferably 90 $\pm$ 15 min;

or preferably, a frequency of administration of the mitoxantrone hydrochloride liposome is once every 3 weeks or 4 weeks.

10. The mitoxantrone hydrochloride liposome as claimed in claim 9, wherein the mitoxantrone hydrochloride liposome has one or more of the following properties: (i) the mitoxantrone hydrochloride liposome has a particle size of about 30-80 nm, e.g., about 35-75 nm, about 40-70 nm, about 40-60 nm, or about 60 nm;

(ii) mitoxantrone hydrochloride forms a poorly soluble precipitate with multivalent counterions in the liposome (e.g., sulfate, citrate, or phosphate);

(iii) a phospholipid bilayer in the mitoxantrone hydrochloride liposome contains a phospholipid with a phase transition temperature (Tm) higher than a body temperature, such that the phase transition temperature of the liposome is higher than the body temperature, for example, the phospholipid is selected from hydrogenated soybean lecithin, phosphatidyl choline, hydrogenated yolk lecithin, dipalmitoyl lecithin, distearoyl lecithin, and any combination of the above;

(iv) the phospholipid bilayer in the mitoxantrone hydrochloride liposome comprises hydrogenated soybean lecithin, cholesterol and polyethylene glycol 2000-modified distearoyl phosphatidyl ethanolamine (DSPE-PEG2000);

(v) the phospholipid bilayer in the mitoxantrone hydrochloride liposome comprises hydrogenated soybean lecithin, cholesterol and polyethylene glycol 2000-modified distearoyl phosphatidyl ethanolamine in a mass ratio of about 3:1:1, mitoxantrone hydrochloride forms a poorly soluble precipitate with multivalent acid ions in the liposome, and the particle size of the mitoxantrone hydrochloride liposome in the drug is about 60 nm;

preferably, the mitoxantrone hydrochloride liposome has a particle size of about 30-80 nm and comprises: 1) mitoxantrone, an active ingredient, which can form a poorly soluble precipitate with multivalent counterions in the liposome, and 2) a phospholipid bilayer containing a phospholipid with a phase transition temperature (Tm) higher than a body temperature, wherein the phospholipid with Tm higher than body temperature is selected from one or more of phosphatidyl choline, hydrogenated soybean lecithin, hydrogenated yolk lecithin, dipalmitoyl lecithin, and distearoyl lecithin;

or preferably, the mitoxantrone hydrochloride liposome has a particle size of about 35-75 nm, preferably 40-70 nm, more preferably 40-60 nm, and even more preferably 60 nm;

or preferably, the phospholipid bilayer comprises hydrogenated soybean lecithin, cholesterol and polyethylene glycol 2000-modified distearoyl phosphatidyl ethanolamine in a mass ratio of 3:1:1, the particle size is about 60 nm, and the counterion is sulfate;

or preferably, the phospholipid bilayer of the liposome comprises hydrogenated soybean lecithin, cholesterol, and polyethylene glycol 2000-modified distearoyl phosphatidyl ethanolamine in a mass ratio of 3:1:1, the particle size is about 40-60 nm, the counterion is sulfate, and in the liposome, a weight ratio of HSPC:Chol:DSPE-PEG2000:mitoxantrone is 9.58:3.19:3.19:1.

## INTERNATIONAL SEARCH REPORT

International application No.

**PCT/CN2022/095446**

**A. CLASSIFICATION OF SUBJECT MATTER**

A61K 9/127(2006.01)i; A61K 31/136(2006.01)i; A61P 35/00(2006.01)i

According to International Patent Classification (IPC) or to both national classification and IPC

**B. FIELDS SEARCHED**

Minimum documentation searched (classification system followed by classification symbols)

A61K A61P

Documentation searched other than minimum documentation to the extent that such documents are included in the fields searched

Electronic data base consulted during the international search (name of data base and, where practicable, search terms used)

DWPI; CNABS; TWABS; CNTXT; EPTXT; USTXT; WOTXT; CNKI; STNext: 盐酸米托蒽醌, 脂质体, 肿瘤, 癌, 石药集团中奇制药技术（石家庄）有限公司, 李春雷, 刘延平, 李彦辉, 夏雪芳, 梁敏, 王彩霞, 李春纳, 张婧婧, 王世霞, 孟红卫, Mitoxantrone hydrochloride, novantrone, liposome, tumor, cancer, CSPC ZHONGQI PHARMACEUTICAL TECHNOLOGY (SHIJIAZHUANG) CO., LIChunlei, LIUYanping, LIYanhui, XIAXuefang, LIANGMin, WAGNCaixia, LIChunna, ZHANGJingjing, WANGShixia, MENGHongwei, 70476-82-3, 65271-80-9

**C. DOCUMENTS CONSIDERED TO BE RELEVANT**

| Category* | Citation of document, with indication, where appropriate, of the relevant passages | Relevant to claim No. |
|---|---|---|
| X | CN 103622909 A (JILIN UNIVERSITY) 12 March 2014 (2014-03-12) claim 9, and description, paragraph 38, and embodiment 6 | 1-10 |
| X | CN 110711178 A (CSPC ZHONGQI PHARMACEUTICAL TECHNOLOGY (SHIJIAZHUANG) CO., LTD.) 21 January 2020 (2020-01-21) description, paragraphs 12, 36, 54, 56, and 74 | 1-10 |
| X | US 2011002977 A1 (LI CHUNLEI et al.) 06 January 2011 (2011-01-06) claims 21-38, and embodiment 2 | 1-10 |
| X | CN 105287383 A (JILIN UNIVERSITY) 03 February 2016 (2016-02-03) claims 1-8, and embodiments 1-2 | 1-10 |
| A | WO 2010103118 A1 (UNIVERSITAETSSPITAL BASEL) 16 September 2010 (2010-09-16) entire document | 1-10 |

☐ Further documents are listed in the continuation of Box C.　☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **16 August 2022** | **25 August 2022** |

| Name and mailing address of the ISA/CN | Authorized officer |
|---|---|
| **China National Intellectual Property Administration (ISA/ CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

| International application No. |
| --- |
| **PCT/CN2022/095446** |

| Box No. II      Observations where certain claims were found unsearchable (Continuation of item 2 of first sheet) |
| --- |

This international search report has not been established in respect of certain claims under Article 17(2)(a) for the following reasons:

1. ☑  Claims Nos.: **7-8**
because they relate to subject matter not required to be searched by this Authority, namely:

   [1]  Claims 7-8 relate to a method for treating advanced solid tumors, and do not comply with PCT Rule
   39.1(iv). A search was conducted on the basis that the title of the subject matter is an application of a
   mitoxantrone hydrochloride liposome in the preparation of drugs for treating advanced solid tumors.

2. ☐  Claims Nos.:
because they relate to parts of the international application that do not comply with the prescribed requirements to such an
extent that no meaningful international search can be carried out, specifically:

3. ☐  Claims Nos.:
because they are dependent claims and are not drafted in accordance with the second and third sentences of Rule 6.4(a).

Form PCT/ISA/210 (continuation of first sheet) (January 2015)

INTERNATIONAL SEARCH REPORT
Information on patent family members

International application No.

**PCT/CN2022/095446**

| Patent document cited in search report | | | Publication date (day/month/year) | Patent family member(s) | | | Publication date (day/month/year) |
|---|---|---|---|---|---|---|---|
| CN | 103622909 | A | 12 March 2014 | None | | | |
| CN | 110711178 | A | 21 January 2020 | AU | 2019301101 | A1 | 28 January 2021 |
| | | | | CN | 112384207 | A | 19 February 2021 |
| | | | | BR | 112021000452 | A2 | 06 April 2021 |
| | | | | EP | 3821887 | A1 | 19 May 2021 |
| | | | | SG | 11202013236 X | A | 25 February 2021 |
| | | | | US | 2021267915 | A1 | 02 September 2021 |
| | | | | CU | 20210003 | A7 | 06 August 2021 |
| | | | | WO | 2020011189 | A1 | 16 January 2020 |
| | | | | JP | 2021530489 | A | 11 November 2021 |
| | | | | CA | 3105698 | A1 | 16 January 2020 |
| | | | | KR | 20210031695 | A | 22 March 2021 |
| US | 2011002977 | A1 | 06 January 2011 | EP | 2123260 | A1 | 25 November 2009 |
| | | | | RU | 2009126983 | A | 10 February 2011 |
| | | | | JP | 2010514708 | A | 06 May 2010 |
| | | | | ES | 2401526 | T3 | 22 April 2013 |
| | | | | JP | 2015180652 | A | 15 October 2015 |
| | | | | CN | 101209243 | A | 02 July 2008 |
| | | | | AU | 2007341803 | A1 | 10 July 2008 |
| | | | | KR | 20090094043 | A | 02 September 2009 |
| | | | | US | 2016235671 | A1 | 18 August 2016 |
| | | | | WO | 2008080367 | A1 | 10 July 2008 |
| | | | | CU | 20090114 | A7 | 24 February 2011 |
| | | | | JP | 2013177397 | A | 09 September 2013 |
| | | | | BR | PI0720733 | A2 | 07 January 2014 |
| | | | | CA | 2673924 | A1 | 10 July 2008 |
| | | | | MY | 150670 | A | 28 February 2014 |
| | | | | IN | 4630DELNP2009 | A | 19 March 2010 |
| | | | | CO | 6190601 | A2 | 19 August 2010 |
| | | | | MX | 2009007089 | A | 08 October 2009 |
| CN | 105287383 | A | 03 February 2016 | None | | | |
| WO | 2010103118 | A1 | 16 September 2010 | EP | 2228059 | A1 | 15 September 2010 |

Form PCT/ISA/210 (patent family annex) (January 2015)

**REFERENCES CITED IN THE DESCRIPTION**

**Patent documents cited in the description**

- CN 202110593815

- WO 2008080367 A1